(19) Europäisches Patentamt
European Patent Office
Office européen des brevets

(11) Publication number: **0 296 463 B1**

(12) # EUROPEAN PATENT SPECIFICATION

(45) Date of publication of patent specification: **10.06.92**

(51) Int. Cl.[5]: **C07D 333/32**, C07D 333/36, C07D 309/22, C07C 45/60, C07C 45/67

(21) Application number: **88109474.2**

(22) Date of filing: **14.06.88**

(54) **Thiophen compounds and their preparation.**

(30) Priority: **16.06.87 GB 8714005**

(43) Date of publication of application:
**28.12.88 Bulletin 88/52**

(45) Publication of the grant of the patent:
**10.06.92 Bulletin 92/24**

(84) Designated Contracting States:
**AT BE CH DE ES FR GB GR IT LI NL**

(56) References cited:
**EP-A- 0 210 320**
**GB-A- 2 114 566**
**US-A- 2 408 518**
**US-A- 2 408 519**
**US-A- 3 980 675**

(73) Proprietor: **SANDOZ AG**
**Lichtstrasse 35**
**CH-4002 Basel(CH)**
(84) Designated Contracting States:
**BE CH ES FR GB GR IT LI NL**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Fünfschilling, Peter**
**Ochsengasse 62**
**CH-4123 Allschwil(CH)**

**EP 0 296 463 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel processes for the preparation of N-(2,4-dimethylthien-3-yl)-N-(1-methoxyprop-2-yl)-chloroacetamide (hereinafter designated thienyl-chloroacetamide) and of intermediates thereof and to novel intermediates useful for the preparation of the thienylchloroacetamide.

UK 2 114 566 discloses the thienyl-chloroacetamide, herbicidal properties of said compound and processes for preparing it. The processes in UK 2 114 566 have various disadvantages. EPA 210320 discloses an improved process of preparing the thienyl-chloracetamide employing better available starting materials. Said process has the ecological disadvantage that it involves the use of thionylchloride as oxydation agent.

The present invention provides novel advantageous processes for the preparation of the thienyl-chloroacetamide and of intermediates therefor. The novel processes allow the preparation of thienyl-chloracetamide in high yields and are ecologically acceptable. The processes involve also novel intermediates.

Accordingly, the invention provides 2,4-dimethyl-2,3-dihydro-thiophen-3-one of formula Ia, which may also exist in its tautomeric enol-form of formula Ib

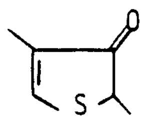

Ia

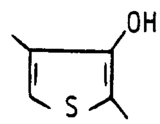

Ib

or salts thereof. The compound of formula Ib may be converted to its salts in a manner known per se or vice versa.

The compound of formula Ia or Ib (hereinafter designated formula I) is obtained by either

a) treating a compound of formula III

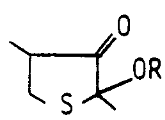

III

wherein R is H, the aliphatic hydrocarbyl moiety of an alkanol, or $C_{2-4}$alkanoyl,
with an organic carboxylic acid in the presence of an amine, or

b) treating a compound of formula IIIb

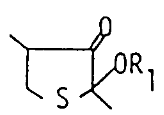

IIIb

wherein $R_1$ is the aliphatic hydrocarbyl moiety of an alkanol,
with NaOH, and converting where desired the thus obtained Na salt of the compound of formula Ib into its free keto/enol form.

Process a) is conveniently effected by treating a solution of the compound of formula III with an alkanecarboxylic acid such as glacial acetic acid, propionic acid and the like. The reaction is effected in the presence of an amine, e.g. a tertiary amine such as triethylamine or a secondary or primary amine such as 1-methoxy-2-propylamine (hereinafter referred to as methoxyisopropylamine). Suitable solvents are solvents which are inert under the reaction conditions, e.g. an hydrocarbon such as n-hexane, toluene, xylene, a chlorinated hydrocarbon such as 1,2-dichloroethane, or mixtures thereof. The compound of formula III may also be and is conveniently prepared in situ and reacted further without isolation. More details on this aspect of the invention will be given below. A suitable reaction temperature for process a) lies in the range of 20 °C - 80 °C, e.g. at 60 °C - 75 °C. The reaction is preferably effected at pH 5 to 12, more preferably at pH 5 - 8.

Process b) is conveniently effected in a solvent which is inert under the reaction conditions, i.e. an

alkanol such as methanol. A suitable reaction temperature for process b) lies in the range of 20 ° C to 80 ° C, e.g. at 60 ° C. The reaction runs smoothly and there is accordingly no need to work at higher temperature. The pH is preferably in the range of 5 -12.

Like in process a), the compound of formula IIIb may be prepared in situ and reacted further without, isolation (see below).

The compound of formula III wherein R is hydrogen may be readily obtained from 4-methyl-4-pentene-2,3-dione of formula IV

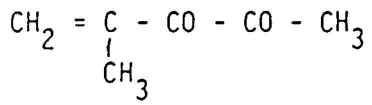

IV

by treatment with $H_2S$ in the presence of a base (process c).

Process c) is essentially a Michael addition and can be effected under the conditions known for such reaction. The reaction is carried out in the presence of an inorganic or organic base, e.g. an alkalimetal hydroxide such as NaOH, or a primary, secondary or tertiary amine, such as methoxyiso propylamine, triethylamine and the like. The reaction requires the presence of only catalytical amounts of base.

The reaction of process c) proceeds already at a temperature of -40 ° C. A suitable reaction temperature is for example from 0 ° to 20 ° C, e.g. from 0 ° to 5 ° C.

The compound of formula IV, $H_2S$ and the base may be and preferably are employed in equimolar amounts.

Process c) is conveniently effected in a solvent which is inert under the reaction conditions, e.g. in a hydrocarbon such as hexane, toluene, xylene, an alkanol such as methanol, a chlorinated hydrocarbon such as 1,2-dichloroethane or mixtures thereof. The $H_2S$ may be introduced in gas form into the reaction mixture.

It is however also possible to add the compound of formula IV to a mixture of $H_2S$ and a base in a solvent which is inert under the reaction conditions.

After the reaction is complete, which can be established by known methods, e.g. chromatographically, the compound of formula III may be converted without further isolation to the compound of formula I, employing process a). The compound of formula III may, of course, also be isolated and then reacted in the next step.

Compounds of formula IIIb are obtained by oxydation of the compound of formula V

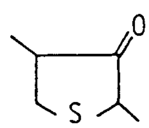

V

with an aqueous solution of a peroxyde, optionally in the presence of an alkanol or a $6_{2-4}$carboxylic acid (process d).

Where process d) is carried out in the absence of an alkanol or a carboxylic acid, the compound of formula III wherein R is H is obtained. (Compound of formula IIIa).

Where process d) is carried out in the presence of an alkanol, a compound of formula III wherein R is the aliphatic hydrocarbyl moiety of an alkanol, is obtained (Compounds of formula IIIb). Examples of alkanols suitable for use in process d) are methanol, ethanol, isopropanol, hexanol; they allow the preparation of compounds of formula IIIb, wherein $R_1$ is $C_{1-6}$alkyl. $R_1$ is particularly $C_{1-4}$alkyl, most preferably methyl.

Where process d) is carried out in the presence of a $C_{2-4}$carboxylic acid, a compound of formula III is obtained wherein R is $C_{2-4}$alkanoyl (Compounds of formula IIIc). Where R is $C_{2-4}$alkanoyl it is preferably acetyl.

An example of an aqueous peroxyde suitable for use in process d) is aqueous $H_2O_2$, e.g. a 35% $H_2O_2$ solution. Reaction d) is exothermic. The reaction temperature lies conveniently between 40 ° C and 80 ° C, e.g. at 60 ° C to 65 ° C.

Compounds of formula IIIb are also obtained by treating the compound of formula IIIa with an aliphatic alkanol in the presence of a strong acid (process e).

In process e) the acid needs only to be present in catalytical amounts. Suitable acids are for example protonating acids such as HCl. Examples of suitable alkanols ($R_1OH$) are methanol, ethanol, isopropanol,

hexanol etc. A suitable procedure is for example to employ the alkanol ($R_1OH$) as solvent.

A suitable reaction temperature is from 20°C to 80°C, e.g. 40°C to 60°C.

The compounds of formula IV and V are known. A suitable process for the preparation of the compound of formula V is disclosed in EPA 210320.

A process for the preparation of the compound of formula IV is disclosed by A. Shabanov et al. in Dokl. Akad. Nauk Azerb. SSR 27(6), 42-46 (1971).

It has been found that the compound of formula IV is, in general, partly obtained in its dimeric form of formula VI

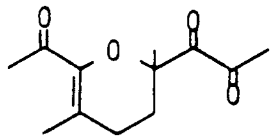

VI

The proportion of dimeric form obtained will depend on the reaction conditions employed for the preparation of the compound of formula IV. It has also been observed that the compound of formula IV dimerizes in undiluted form at room temperature (though said compound can be stored in a hydrocarbon such as hexane or toluene).

It has now been found that the monomeric compound of formula IV may be obtained by pyrolysis of the compound of formula VI (process f), and this in high yields.

A suitable reaction temperature for process f) is between 400°C and 600°C at a pressure of 0.01 to 100 Torr, e.g. at 4 to 8 Torr. The compound of formula VI may be distilled under the aforementioned conditions, e.g. in a quartz tube.

The compound of formula VI is conveniently obtained by elimination of HBr from the compound of formula VII

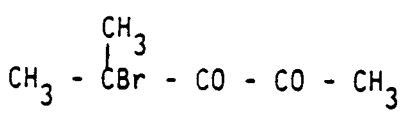

VII

in the presence of an acid binding agent (process g).

Said process g) may be effected under the conditions known for the preparation of olefines from halogenated aliphatic ketones. The reaction is conveniently carried out in a solvent which is inert under the reaction condition for example an aliphatic or aromatic hydrocarbon such as hexane or xylene. The reaction temperature is preferably above room temperature, e.g. in the range of 100°C to 130°C when employing xylene as a solvent. Examples of suitable acid binding agents are tertiary alkylamines, such as $N(nC_4H_9)_3$. As indicated earlier, part of the compound of formula IV will directly dimerize under the reaction conditions to the compound of formula VI; the latter compound may then be converted to the compound of formula IV by pyrolysis.

The reaction of the compound of formula I with 1-methoxy-2-propylamine allows the production of the N-(1-methoxyprop-2-yl)-2,4-dimethylaminothiophene in high yields (process h).

Such process h) is conveniently effected in an autoclave. 1-Methoxy-2-propylamine may thereby serve as solvent. The reaction is advantageously carried out in the presence of an acid such as hydrochloric acid. The reaction temperature is preferably above 100°C, e.g. between 150°C and 220°C.

Reaction of N-(1-methoxyprop-2-yl)-2,4-dimethylaminothiophene with chloroacetylchloride, under the conditions analogous to those of the process of EPA 210320, yield then the desired thienyl-chloroacetamide.

The invention may be illustrated by the following examples in which temperatures are in Centigrades.

Example 1 : 2,4-Dimethyl-2,3-dihydrothiophen-3-one and 2,4-dimethyl-3-hydroxythiophene

**a.** 2,4-Dimethyl-2-hydroxy-tetrahydro-thiophen-3-one

Into a solution of 224 mg (0.20 mmol) of 4-methyl-4-pentene-2,3-dione and 202 mg (0.20 mmol) of triethylamine in 5 ml of n-hexane and 3 ml of 1,2-dichloroethane are introduced within 1 hour and at 0-5°

internal temperature, 70 mg of $H_2S$ gas. After said hour it is no longer possible to determine the starting material (dione) gaschromatographically. The clear solution, comprising 2.4-dimethyl-2-hydroxy-tetrahydrothiophen3-one may now, without isolation of the hydroxyketone, be further treated as shown in step b) hereinafter.
The hydroxyketone may also be isolated as follows: the reaction solution is eluated over silica gel employing toluene/ethylacetate 9:1 as eluant, the solvent is evaporated off and the residue distilled in a bulb tube oven. The hydroxyketone (subtitle a) distills at 55° and 0.01 Torr (light yellow oil; according to NMR in the form of a 1:1-cis/trans mixture).

**b.** Title Compounds (Example 1)

To the reaction solution of Example 1a) are added 0.2 ml acetic acid and the mixture is stirred for 15 hours at 60° (internal temperature). The mixture is concentrated in vacuo and the residue distributed between 5 ml hexane and 5 ml 1N sodium hydroxide. The organic phase is separated and removed.
The aqueous phase is covered with 5 ml hexane and 3 ml saturated ammonium chloride solution are added. The phases are separated and the aqueous phase extracted one more time with 5 ml hexane. The combined organic phases are concentrated in vacuo. The remaining title compound consists according to NMR determination of a tautomeric mixture comprising 60 % keto compound and 40 % enol compound.

Example 2 : 2,4-Dimethyl-2,3-dihydrothiophen-3-one and 2,4-dimethyl-3-hydroxythiophene

**a.** 2,4-Dimethyl-2-methoxy-tetrahydrothiophen-3-one

To a solution of 411 g (3.16 mol) of 2,4-dimethyl-tetrahydro-thiophen-3-onein 3000 ml of methanol are added dropwise, at an internal temperature of 63-65° and over a period of one hour, 256.4 g (2.70 mol) of 35.8 % hydrogen peroxide..
The reaction is exothermic; from time to time it is necessary to remove the heating bath. After the addition is completed the reaction mixture is stirred for another 2 hours at the internal temperature of 63-65°. The reaction mixture comprises, according to gas chromatographical determination - besides some 2,4-dimethyl-2-hydroxy-tetrahydrothiophene-3-one mainly 2,4-dimethyl-2-methoxy-tetrahydrothiophen-3-one.

**b.** 2,4-Dimethyl-2,3-dihydrothiophen-3-one and 2,4-dimethyl-3-hydroxythiopene

The reaction mixture of Example 2a is cooled to 60° and then 240 ml of an aqueous 30 % (2,43 mol) sodium hydroxide solution are added. After 15 minutes stirring at 60° the reaction mixture has turned violet. It contains according to the gas chromatogram no more 2,4-dimethyl-2-methoxy-tetrahydrothiophen-3-one.
The reaction is then concentrated in vacuo. The residue is diluted with 1500 ml of water and non-phenolic compounds are removed by extraction with cyclohexane (3 x 300 ml). The aqueous phase is covered with 300 ml cyclohexane and, at an internal temperature of 20-25°, adjusted at pH = 9.0 by dropwise addition of 205 ml (1.97 mol) of conc. hydrochloric acid. The phases are separated, and the aqueous phase is extracted again with cyclohexane (4 x 300 ml). The combined organic phases are concentrated in vacuo and the residue is distilled under low absolute pressure: the main fraction distills over at 53-60° and 0.02 Torr, as a light yellow oil, consisting according to NMR of a tautomeric mixture of the title compound comprising ca. 60 % of the keto compound and 40 % of the enol compound.

Example 3: 2,4-Dimethyl-2,3-dihydrothiophene-3-one and 2,4-dimethyl-3-hydroxythiophene

**a.** 2,4-Dimethyl-2-methoxy-tetrahydrothiophen-3-one

To a solution of 261 g (2.0 mol) of 2,4-dimethyl-tetrahydrothiophen-3-one and 6 g of acetic acid in 600 ml of methanol are added dropwise, at an internal temperature of 20-25° (external cooling with ice/water) over a period of 3 hours 207 g (2.1 mol) of 34.5 % hydrogen peroxide. The reaction mixture is then stirred over night at 20-25°. According to gas chromatographical determination the reaction mixture comprises -beside some 2,4-dimethyl-2-hydroxy-tetrahydrothiophene-3-one (compound IIIa) - mainly 2,4-dimethyl-2-methoxy-tetrahydrothiophene-3-one.

**b.** 2,4-Dimethyl-2,3-dihydrothiophen-3-one and 2,4-dimethyl-3-hydroxythiophene

By adding 5 ml of 30 % NaOH the pH of the reaction mixture is adjusted from 2.5 to 6.2. The methanol is distilled off at ca. 900 mbar (internal temperature = 74°, duration = 3 hours). During this distillation both compound IIIa and compound IIIb ($R_1$ = $CH_3$) are transformed to the keto/enol mixture Ia and Ib.
To the mixture (now two phases) are added 500 ml of toluene and 100 ml of water. After separation the organic layer is washed with 100 ml of water and the combined aqueous phases are extracted with 100 ml of toluene. The toluene is removed by distillation. 269 g of a dark yellow oil (269 g) are obtained which is distilled at 30 mmHg, b.p. = 110-115°. Yield = 214 g. Purity (according to gas chromato graphical determination) is 95.2 % by weight .

Example 4 : N-(1-methoxyprop-2-yl)-2,4-dimethyl-3-aminothiophene

In a 200 ml autoclave are heated, at 190°, with stirring 12.8 g (0.10 mol) of the 60 : 40 mixture of 2,4-dimethyl-2,3-dihydrothiophen-3-one and 2,4-dimethyl-3-hydroxythiopene, 100 ml of 1-methoxy-2-propylamine and 8.5 ml conc. hydrochloric acid. The pressure rises thereby to maximum 9 bar.

The reaction mixture is worked up by adding 15 ml of 30 % sodium hydroxide solution and removal of the excess of 1-methoxy-2-propylamine in vacuo.

To the evaporation residue are added 100 ml of water and 100 ml of cyclohexane and the phases separated in a separating funnel. The aqueous phase is extracted again with cyclohexane (3 x 50 ml).

To the combined organic phases are added 50 ml of water. Then 15 ml of conc. hydrochloric acid are added to adjust the organic solution at pH = 1. The phases are separated and the organic phase washed with water (2 x 50ml.

The combined aqueous phases are covered with 100 ml of cyclohexane and adjusted at pH = 13 with 22 ml of 30 % sodium hydroxide solution. After separation with a separating funnel the aqueous phases are again extracted with cyclohexane (2 x 50 ml). The combined organic phases are completely concentrated to give the title compound having a gas chromatographical purity of 96.3 %.

Example 5 : 4-Methyl-4-pentene-2,3-dione

10.0 g (0.089 mol) of 6-acetyl-2,5-dimethyl-2-(1,2-dioxo-propyl)-2H-3,4-dihydropyran is distilled at 6 Torr through a quartz tube which was preheated at 500° to give the title compound.

Example 6 : 6-Acetyl-2,5-dimethyl-2-(1,2-dioxo-propyl)-2H-3,3-dihydropyran
(Compound of formula VI - process f)

A mixture of 60.0 g (0.31 mol) of 4-bromo-4-methyl-pentane-2,3-dione, and 0.1 g hydroquinone in 300 ml of tri-(n-butyl)amine and 1200 ml of xylene is stirred for 10 hours at 130° internal temperature. The reaction mixture contains then, according to chromatographical determination, a mixture of 10 % of 4-methyl-4-pentene-2,3-dione and 90 % of the title compound.

100 ml of conc. hydrochloric acid are then added while cooling the reaction mixture with ice. The phases are separated. The organic phase is washed with water (3 x 250 ml) and completely concentrated in vacuo. The residue is distilled to give the title compound (b.p. 102-106° at 6 Torr) as a light yellow oil.

**Claims**

**1.** Process of preparing 2,4-dimethyl-2,3-dihydro-thiophen-3-one of formula Ia/Ib

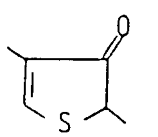 Ia 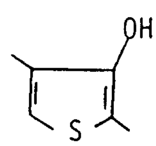 Ib

which comprises either

a) treating a compound of formula III

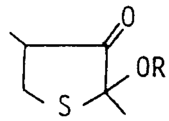

III

wherein R is H, the aliphatic hydrocarbyl moiety of an alkanol or $C_{2-4}$alkanoyl, with an organic carboxylic acid in the presence of an amine, or
b) treating a compound of formula IIIb

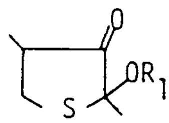

IIIb

wherein $R_1$ is the aliphatic hydrocarbyl moiety of an alkanol, with NaOH, and converting where desired the thus obtained Na salt of the compound of formula Ib into its free keto/enol form.

2. Process of preparing 2,4-dimethyl-2-hydroxy-tetrahydrothiophen-3-one (the compound of formula III stated in Claim 1 wherein R is hydrogen) which comprises treating 4-methyl-4-pentene-2,3-dione with $H_2S$ in the presence of a base.

3. Process of preparing a compound of formula IIIb stated in Claim 1, comprising either
a) etherifying a compound of formula III stated in Claim 1, wherein R is hydrogen with an aliphatic alkanol $R_1OH$, wherein $R_1$ is as defined in Claim 1, or
b) oxydizing the compound of formula V

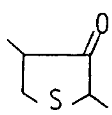

V

with a peroxyde in the presence of an alkanol $R_1OH$, in which $R_1$ is as defined in Claim 1.

4. Process of preparing N-(2,4-dimethylthien-3-yl)-N-(1-methoxyprop-2-yl)-chloracetamide comprising the steps of
a) preparing N-(1-methoxyprop-2-yl)-2,4-dimethyl-3-aminothiophene and
b) N-chloroacetylating N-(1-methoxyprop-2-yl)-2,4-dimethyl-3-aminothiophene with chloroacetylchloride,
characterized in that N-(1-methoxyprop-2-yl)-2,4-dimethyl-3-aminothiophene is obtained by reaction of the compound of formula Ia, Ib or a mixture thereof with 1-methoxy-2-propylamine.

5. The compounds of formulae Ia and Ib, stated in Claim 1.

6. A compound of formula III stated in Claim 1, wherein R is as stated in Claim 1.

7. The compound of Claim 6, wherein R is H.

8. The compound of Claim 6, wherein R is $C_{1-6}$alkyl.

9. The compound of Claim 6, wherein R is $C_{2-4}$alkanoyl.

## Revendications

1. Procédé de préparation de la 2,4-diméthyl-2,3-dihydro-thiophène-3-one de formule Ia/Ib

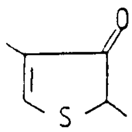 Ia 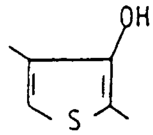 Ib

qui comprend

a) le traitement d'un composé de formule III

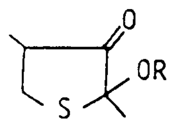 III

dans laquelle R signifie H, le reste hydrocarbyle aliphatique d'un alcanol ou un groupe alcanoyle en $C_2$-$C_4$,

par un acide carboxylique organique en présence d'une amine, ou

b) le traitement d'un composé de formule IIIb

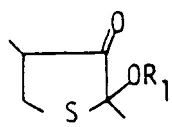 IIIb

dans laquelle $R_1$ signifie le reste hydrocarbyle aliphatique d'un alcanol,

par NaOH et, si nécessaire, la transformation du sel de sodium ainsi obtenu du composé de formule Ib en sa forme céto/énol libre.

**2.** Procédé de préparation de la 2,4-diméthyl-2-hydroxy-tétrahydrothiophène-3-one (le composé de formule III spécifié à la revendication 1 dans lequel R signifie l'hydrogène), qui comprend le traitement de la 4-méthyl-4-pentène-2,3-dione par $H_2S$ en présence d'une base.

**3.** Procédé de préparation d'un composé de formule IIIb spécifié à la revendication 1, qui comprend

a) l'éthérification d'un composé de formule III spécifié à la revendication 1, dans lequel R signifie l'hydrogène, avec un alcanol aliphatique $R_1OH$, dans lequel $R_1$ est tel que défini à la revendication 1, ou

b) l'oxydation du composé de formule V

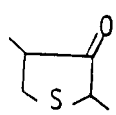 V

avec un péroxyde en présence d'un alcanol $R_1OH$, dans lequel $R_1$ est tel que défini à la revendication 1.

**4.** Procédé de préparation du N-(2,4-diméthyl-3-thiényl)-N-(1-méthoxy-2-propyl)-chloroacétamide comprenant les étapes

a) de préparation du N-(1-méthoxy-2-propyl)-2,4-diméthyl-3-aminothiophène et

b) la N-chloroacétylation du N-(1-méthoxy-2-propyl)-2,4-diméthyl-3-aminothiophène avec du chlorure de chloroacétyle,

caractérisé en ce que le N-(1-méthoxy-2-propyl)-2,4-diméthyl-3-aminothiophène est obtenu par réaction du composé de formule Ia, Ib ou un mélange de ces composés, avec la 1-méthoxy-2-propylamine.

**5.** Les composés de formule Ia et Ib, spécifiés à la revendication 1.

6. Un composé de formule III spécifié à la revendication 1, dans lequel R est tel que spécifié à la revendication 1.

7. Le composé de la revendication 6, dans lequel R signifie H.

8. Le composé de la revendication 6, dans lequel R signifie un groupe alkyle en $C_1$-$C_6$.

9. Le composé de la revendication 6, dans lequel R signifie un groupe alcanoyle en $C_2$-$C_4$.

**Patentansprüche**

1. Verfahren zur Herstellung von 2,4-Dimethyl-2,3-dihydro-thiophen-3-on der Formel Ia/Ib

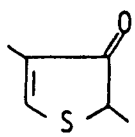 Ia 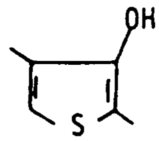 Ib

umfassend, daß man entweder
a) eine Verbindung der Formel III

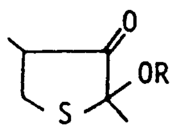 III

worin R H, der aliphatische Kohlenwasserstoffrest eines Alkanols oder ein $C_2$-$C_4$-Alkanoylrest ist, mit einer organischen Carbonsäure in Gegenwart eines Amins behandelt, oder
b) eine Verbindung der Formel IIIb

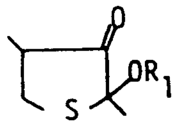 IIIb

worin $R_1$ der aliphatische Kohlenwasserstoffrest eines Alkanols ist, mit NaOH behandelt und, falls erwünscht, das so erhaltene Na-Salz der Verbindung der Formel Ib in die freie Keto/Enol-Form umwandelt.

2. Verfahren zur Herstellung von 2,4-Dimethyl-2-hydroxy-tetrahydrothiophen-3-on (Verbindung der Formel III, wie in Anspruch 1 angegeben, worin R Wasserstoff ist), umfassend, daS man 4-Methyl-4-penten-2,3-dion mit $H_2S$ in Gegenwart einer Base behandelt.

3. Verfahren zur Herstellung einer Verbindung der Formel IIIb, wie in Anspruch 1 angegeben, umfassend, daß man entweder
a) eine Verbindung der Formel III, wie in Anspruch 1 angegeben, worin R Wasserstoff ist, mit einem aliphatischen Alkanol $R_1OH$, worin $R_1$ wie in Anspruch 1 definiert ist, verethert, oder
b) die Verbindung der Formel V

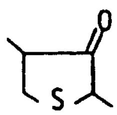 V

mit einem Peroxid in Gegenwart eines Alkanols $R_1OH$, worin $R_1$ wie in Anspruch 1 definiert ist, oxidiert.

**4.** Verfahren zur Herstellung von N-(2,4-Dimethylthien-3-yl)-N-(1-methoxyprop-2-yl)-chloracetamid, umfassend die Schritte, daß man

a) N-(1-Methoxyprop-2-yl)-2,4-dimethyl-3-aminothiophen herstellt und

b) N-(1-Methoxyprop-2-yl)-2,4-dimethyl-3-aminothiophen mit Chloracetylchlorid N-chloracetyliert,

**dadurch gekennzeichnet,** daß N-(1-Methoxyprop-2-yl)-2,4-dimethyl-3-aminothiophen erhalten wird durch die Reaktion der Verbindung Ia, Ib oder einer Mischung davon mit 1-Methoxy-2-propylamin.

**5.** Verbindungen der Formeln Ia und Ib, wie in Anspruch 1 angegeben.

**6.** Verbindung der Formel III, wie in Anspruch 1 angegeben, worin R wie in Anspruch 1 angegeben ist.

**7.** Verbindung nach Anspruch 6, worin R H ist.

**8.** Verbindung nach Anspruch 6, worin R ein $C_1$-$C_6$-Alkylrest ist.

**9.** Verbindung nach Anspruch 6, worin R ein $C_2$-$C_4$-Alkanoylrest ist.